# EUROPÄISCHE PATENTSCHRIFT

(11) **EP 1 389 908 B1**
(45) Veröffentlichungstag und Bekanntmachung des Hinweises auf die Patenterteilung: **13.06.2012**
(21) Anmeldenummer: 02732710.5
(22) Anmeldetag: 11.05.2002
(51) Int. Cl.: A01N 37/16, A61L 2/18, A61L 2/10

(54) **ENTKEIMUNG VON OBERFLÄCHEN**
STERILIZATION OF SURFACES
STERILISATION DE SURFACES

(30) Priorität: 21.05.2001 DE 10124817
(43) Veröffentlichungstag der Anmeldung: 25.02.2004
(73) Patentinhaber: ECOLAB INC., St. Paul, MN 55102-2233 (US)
(72) Erfinder: BIERING, Holger, 41516 Grevenbroich (DE); GLASMACHER, Rudolf, 40789 Monheim (DE); VON RHEINBABEN, Friedrich, 40789 Monheim (DE)
(74) Vertreter: Polypatent
(86) Internationale Anmeldenummer: PCT/EP2002/005199
(87) Internationale Veröffentlichungsnummer: WO 2002/102154

(56) Entgegenhaltungen:
- EP-A- 0 027 278
- EP-A- 0 411 970
- EP-A- 0 572 975
- EP-A- 0 818 206
- EP-A- 1 095 664
- WO-A-01/78793
- WO-A-80/01457
- WO-A-92/18170
- WO-A-96/05869
- WO-A-97/06106
- US-A- 4 448 750
- US-A- 6 103 189
- US-B1- 6 187 266
- C. LUBELLO, C. CARETTI AND R. GORI: "Comparison between PAA/UV and H2O2/UV disinfection for wastewater reuse" WATER SCIENCE AND TECHNOLOGY: WATER SUPPLY, Bd. 2, Nr. 1, 2002, Seiten 205-212, XP001096274

## Beschreibung

Die vorliegende Erfindung umfaßt die Verwendung eines Mittels, erhältlich durch künstliche UV-Bestrahlung zur sterilisierenden Behandlung von medizinischen Instrumente ohne Sterilwasser-Nachspülung.

Die US 4,731,222 sowie die US 4,892,706 beschäftigen sich mit der Sterilisation medizinischer Instrumente unter Einsatz von flüssigen Desinfektionsmitteln. Dabei werden die medizinischen Instrumente durch Spülen mit Sterilwasser, erzeugt durch Filtration von beispielsweise Leitungswasser über ein Sterilfilter, von den Rückständen des Desinfektionsmittels freigespült.

Die US 6,103,189 offenbart ein Verfahren, bei dem auf Sterilwasser als Nachspülwasser verzichtet wird, indem man mit einer sterilisierenden Lösung nachspült, die frei von nicht zerfallenden Komponenten ist. Dabei sind gemäß dem Beispiel in Spalte 5, Zeile 16 bis 18, die Bedingungen so zu wählen, daß eine Sterilisation des verwendeten Leitungswassers erreicht wird.

EP 041197011 beschreibt die Sterilisierung von plastikcontainern für hebensmittel durch Einsatz von H₂O₂, Persäuren und UV strahlung.

Die im Stand der Technik vorliegenden Verfahren zur sterilisierenden Behandlung medizinischer Instrumente beschäftigen sich im wesentlichen mit dem Einsatz chemischer Desinfektionsmittel zur Abtötung der relevanten Mikroorganismen. Der Nachteil der bekannten Technologien ist, daß teilweise hoher Aufwand hinsichtlich der notwendigen Zeit und den für eine ausreichende Wirksamkeit erforderlichen Mengen an Desinfektionsmitteln betrieben werden muß. Diese hohen Einsatzmengen an Desinfektionsmittel bei der sterilisierenden Behandlung medizinischer Instrumente bzw. zur Konditionierung der für die Nachspülung vorgesehenen Flüssigkeit führt zu gravierenden Problemen. Je größer die auf der Oberfläche der medizinischen Instrumente zurückbleibende Menge an Desinfektionsmittel ist, desto größer ist die Menge an Desinfektionsmittel, die bei einer späteren Anwendung der medizinischen Instrumente mit dem jeweiligen Patienten in Berührung gebracht wird. Andererseits kann die einzusetzende Menge nicht beliebig gesenkt werden, da, wie auch in den Beispielen der vorliegenden Erfindung gezeigt wird, Wirkungslücken bei wichtigen Keimspektren auftreten können, die in der Praxis, speziell im medizinischen Bereich, nicht akzeptabel sind.

Dementsprechend war es Aufgabe der vorliegenden Erfindung, Mittel, Verfahren und Systeme zur Verfügung zu stellen, die es ermöglichen, die Menge der für die sterilisierende Behandlung erforderlichen chemischen Desinfektionsmitteln und insbesondere die auf den Oberflächen nach Beendigung der Behandlung zurückbleibende Menge chemische Desinfektionsmittel zu reduzieren und dabei noch eine ausreichende Wirksamkeit gegen die relevanten Keime zu erreichen.

Gegenstand der vorliegenden Erfindung ist die Verwendung nach Anspruch 1.

Besonders bevorzugt sind erfindungsgemäße Mittel, bei denen die genannten organischen Persäuren ausgewählt sind aus
a) den Persäuren oder Salzen von Persäuren mit der allgemeinen Formel I

   R²-O₂C-(CH₂)ₓ-CO₃H (I)

   worin R² Wasserstoff oder eine Alkylgruppe von 1 bis 4 Kohlenstoffatomen und
   x eine Zahl von 1 bis 4 ist, und/oder
b) den Phthalimido-Percarbonsäuren (II), worin der Percarbonsäure-Anteil 1 bis 18 Kohlenstoffatome enthält, und/oder
c) den Verbindungen der Formel III

   R¹-CO₃H (III)

   worin R¹ eine Alkyl- oder Alkenylgruppe mit 1 bis 18 Kohlenstoffatomen ist.

Dabei ist es ganz besonders bevorzugt, daß in dem erfindungsgemäßen Mittel
a) als Persäuren gemäß der allgemeinen Formel I Persäuren enthalten sind, in denen R² Wasserstoff oder eine Methylgruppe ist, und/oder
b) als Persäuren Phthalimido-Persäuren enthalten sind, in denen der Percarbonsäure-Anteil 1 bis 8 Kohlenstoffatome enthält, und/oder
c) als Persäuren gemäß der allgemeinen Formel III Persäuren mit einer Alkyl-oder Alkenylgruppe mit 1 bis 12 Kohlenstoffatomen enthalten sind.

In einer überaus bevorzugten Ausführungsform der vorliegenden Erfindung enthält das erfindungsgemäße Mittel als Persäure eine oder mehrere Verbindungen ausgewählt aus Peressigsäure, Perpropionsäure, Peroctansäure, Phthalimidoperhexansäure, Phthalimidoperoctansäure, Persuccinsäure, Persuccinsäuremonomethylester, Perglutaräsure, Perglutarsäuremonometylester, Peradipinsäure, Peradipinsäuremonomethylester, Perbernsteinsäure, Perbernsteinsäuremonomethylester, wobei aus der Auswahl dieser Komponenten die Peressigsäure hervorzuheben ist. Für den Fall, daß das erfindungsgemäße Mittel nicht dafür vorgesehen ist, im letzten Nachspülschritt eingesetzt zu werden, enthält das erfindungsgemäße Mittel vorzugsweise zusätzlich Fettsäuren mit 8 bis 12 Kohlenstoffatomen und/oder anionische Tenside ausgewählt aus den C₈-C₁₈-Alkylsulfaten, C₈-C₁₈-Alkylethersulfaten, C₈-C₁₈-Alkansulfonaten, C₈-C₁₈-α-Olefinsulfonaten, sulfonierten C₈-C₁₈-Fettsäuren, C₈-C₁₈-Alkylbenzolsulfonaten, Sulfonbernsteinsäuremono- und -di-C₁-C₁₂-Alkylestern, C₈-C₁₈-Alkylpolglykolethercarboxylaten, C₈-C₁₈-N-Acyltauriden, C₈-C₁₈-N-Sarkosinaten, C₈-C₁₈-Alkylisethionaten sowie Gemischen der voranstehenden.

Es ist ebenfalls bevorzugt, das erfindungsgemäße Mittel durch UV-Bestrahlung einer Lösung, die, abgesehen von Wasser und den Zerfallsprodukten des genannten in der Lösung enthaltenen Desinfektionsmittels, frei von nicht zerfallenden Bestandteilen ist, zu erhalten. Dabei sind als Bestandteile des Mittels im Sinne der Erfindung solche zu verstehen, die absichtlich zugesetzt werden und unter den beschriebenen Bedingungen stabil bleiben. Demgemäß sind in diesem Zusammenhang unabsichtlich zugesetzte Bestandteile, wie beispielsweise Salze, die bei Verwendung von Leitungswasser in das Mittel gelangen, nicht zu berücksichtigen. Nicht zerfallende Bestandteile liegen auch nach einem wesentlichen Zeitraum, beispielsweise nach einem Tag oder nach einer Woche noch in der Ausgangskonzentration vor. Im Gegensatz dazu stehen gemäß vorliegender Erfindung Substanzen, die beispielsweise durch Disproportionierung oder aufgrund ihres oxidativen Charakters nach und nach zerfallen und deren Spaltprodukte entweder weniger antimikrobiell bzw. toxisch oder zumindest so instabil sind, daß sie schnell mit anderen Komponenten zu Zerfallsprodukten reagieren, die weniger antimikrobiell bzw. toxisch sind als die Ausgangssubstanz. Wenn im Rahmen der vorliegenden Erfindung von Mitteln oder Lösungen gesprochen wird, die frei von nicht zerfallenden Inhaltsstoffen sind, sind davon grundsätzlich Wasser und die Zerfallsprodukte der in den genannten Mitteln oder Lösungen enthaltenen Desinfektionsmittel ausgenommen.

### Beispiele

### Durchführung:

Zur Durchführung der mikrobiologischen Untersuchungen wurde im Rahmen der vorliegenden Erfindung mit einer Desinfektionsmittellösung DES1 gearbeitet, die rezepturgemäß eine Mischung von 61,1 Gew.-% vollentsalztem Wasser, 28,7 Gew.-% Wasserstoffperoxid und 10,2 Gew.-% Essigsäure darstellt. Der resultierende Anteil an Peressigsäure liegt in der DES1 bei 4,5 Gew.-%, bezogen auf die gesamte DES1.

Als Testkeime wurden im Rahmen der Versuche Pseudomonas aeruginosa (DSM 939) und Bacillus subtilis (ATCC6633), Sporensuspension verwendet.

Das zu verwendende Wasser wurde mit den Prüfkeimen in folgenden Konzentrationen kontaminiert:

| | |
|---|---|
| Pseudomonas aeruginosa | ca. 10⁴ - 10⁵ KBE/ml |
| Bacillus subtilis | ca. 10³ - 10⁴ KBE/ml |

Sofern nur mit einer der genannten Keimarten kontaminiert wurde, wird darauf im folgenden speziell hingewiesen.

Der Versuch wurde bei eingeschalteter und eingebrannter UV-Anlage (Vorlaufzeit 30 Min.) zunächst in einem Durchgang mit kontaminiertem Wasser ohne DES1 und danach mit kontaminiertem Wasser und soviel DES1 durchgeführt, daß in der Lösung in einem Versuch etwa 30 und in einem anderen Versuch etwa 10 ppm Peressigsäure vorlagen. Die Anlage wurde zwischen jedem Durchgang mit Wasser gespült und desinfiziert, damit gegebenenfalls zurückgebliebene Prüfkeime das Ergebnis des nächsten Durchgangs nicht verfälschen konnten.

Als UV-Anlage wurde eine zur Desinfektion von flüssigen Mechén im Durchflußverfahren geeignete UV-Anlage eingesetzt. Dabei wird durch Quecksilber-Niederdruck-Strahler künstliche UV-C-Strahlung erzeugt. Auf der Wellenlänge von 254 nm beträgt der Anteil ca. 80 % der gesamten Strahlung eines UV-Strahlers (die Absorptionskurve von DNA weist bei 260 nm ein Maximum auf). Die Durchflußleistung durch die genannte UV-Anlage liegt vorzugsweise zwischen 640 und 800 l/h.

Die tatsächliche Peressigsäure-Konzentration wurde nach regelmäßiger Probenahme über gängige Methoden bestimmt. Diese tatsächlich bestimmte Peressigsäurekonzentration ist in den Tabellen 1 und 2 in Klammern enthalten.

### Es wurden außerdem folgende Versuche durchgeführt:

Durchführung eines Versuchsdurchgangs mit kontaminiertem Wasser (nur mit Pseudomonas aeruginosa) bei abgeschalteter UV-Anlage und ohne DES1-Dosierung.

Durchführung mit abgeschalteter UV-Anlage unter Verwendung von DES1 in Mengen, die in einem Versuch zu ca. 30 ppm und in einem anderen Versuch zu einer Konzentration von ca. 10 ppm Peressigsäure in dem kontaminiertem Wasser führen (nur Pseudomonas aeruginosa). Zwischen jeder DES1-Dosierung wurde auch bei diesen Versuchen die Anlage desinfiziert.

Durchführung eines Versuchsdurchgangs mit kontaminiertem Wasser (nur mit Pseudomonas aeruginosa) bei eingeschalteter UV-Anlage, jedoch ohne Dosierung von DES1.

### Zur Probenahme:

Bei der Probenentnahme erfolgte die Entnahme einer ausreichenden Menge Wasser, Neutralisation des Wirkstoffes mittels Inaktivierungs-Lösung, anschließende Membran-Filtration sowie das Anfertigen einer Verdünnungsreihe und Keimzahlbestimmung.

### Ergebnisse:

Der Prüfkeim Pseudomonas aeruginosa, der gleichzeitig zu den wichtigsten Wasserkeimen gehört, wird in der Anlage in Gegenwart des Produkts DES1 deutlich reduziert. Durch Einschalten der UV-Anlage kann die für die Keimreduktion erforderliche Konzentration von DES1 wesentlich reduziert werden. Die Ergebnisse sind den Tabellen zu entnehmen. Die UV-Behandlung allein führt bei diesem Prüfkeim hingegen nur zu einer leichten Keimreduktion.

Die Keimzahl des Testkeims Bacillus subtilis wird bereits durch die UV-Behandlung deutlich reduziert. Hier zeigt DES1 allein kaum eine Wirkung. Jedoch ist die Keimreduktion in den Versuchen mit UV in Gegenwart von DES1 wesentlich größer als in Versuchen mit UV-Behandlung ohne DES1.

Über die Gesamtheit beider eingesetzter Testkeime ist mit der kombinierten Anwendung von DES1 in Verbindung mit UV-Licht eine gute Wirksamkeit zu erzielen. Weder durch DES1 noch durch UV-Bestrahlung alleine können derart vorteilhafte Ergebnisse erreicht werden. Es konnte auch nicht erwartet werden, daß durch das Zusammenwirken von UV-Licht und DES1 diese überraschende Wirkungssteigerung auftritt. Insbesondere ist festzustellen, daß es möglich ist, die Einsatzmengen von DES1, die zur vollen Abtötung beider Testkeime erforderlich sind, durch zusätzliche Behandlung mit UV-Bestrahlung wesentlich zu reduzieren. Diese Tatsache ist besonders wichtig, da es dadurch gelingt, die Konzentration an Peressigsäure unter der Grenze von 50 ppm zu halten, über der erfahrungsgemäß der Geruch von Essigsäure/Peressigsäure bei der Anwendung für die Beschäftigten störend ist. Abgesehen davon ist die Möglichkeit zur Absenkung der Konzentration antimikrobieller Substanzen auch aus toxikologischer Sicht zu begrüßen. Dementsprechend wird bei Einsatz von Mitteln, Verfahren und Systemen, die dem erfindungsgemäßen Prinzip folgen, erreicht, daß die Rückstandsmenge von evtl. toxikologisch kritischen Substanzen sehr gering ist. Demzufolge verringert sich auch das Risiko in der Anwendung.

**Tabelle 1:**

| **Prüfung der Keimreduktion unter Einfluß einer Peressigsäurelösung mit und ohne UV-Bestrahlung** | | |
|---|---|---|
| **Versuchsanordnung** | | |
| **Keimgehalt nach der jeweiligen Behandlung** | **Pseudomonas aeruginosa DSM 939 (K 1111)** | **Bacillus subtilis (ATCC 6633) Sporensuspension** |
| Keimbelastung in der Anlage vor der Behandlung | 5,6 x 10⁴/ml | 6,4 x 10⁴/ml |
| nur UV-Bestrahlung | 4,86 x 10³/ml | 9,7 x 10¹/ml |
| UV plus DES1 | 12 KBE / 100 ml | 56 KBE/100 ml* |
| | (28 ppm PES) | (28 ppm PES) |
| DES1 ohne UV | 2,5 x 10⁰/ml | 7,5 x 10⁴/ml |
| | (31 ppm PES) | (31 ppm PES) |
| UV plus DES1 | 1 KBE/100 ml* | 37 KBE/100 ml* |
| | (12 ppm PES) | (12 ppm PES) |
| DES1 ohne UV | 5,70 x 10²/ml | 7,3 x 10⁴/ml |
| | (11,5 ppm PES) | (14 ppm PES) |

| | | |
|---|---|---|
| *) Die Angaben "pro 100 ml" beziehen sich auf Versuchsergebnisse, wo auch nach Aufarbeitung von 100 ml nur die angegebene Anzahl Keime nachgewiesen wurde. | | |

## Patentansprüche

1. Verwendung eines Mittels, erhältlich durch künstliche UV-Bestrahlung einer wässrigen Lösung, die 0,0001 bis 0,005 Gew.-% chemisches Desinfektionsmittel bezogen auf die gesamte Lösung enthält, zur sterilisierenden Behandlung medizinischer Instrumente, unter Verzicht auf Nachspülung mit reinem Sterilwasser, wobei
a) die medizinischen Instrumente mit einem ersten Mittel gemäß der vorstehenden Definition dekontaminiert werden und danach
b) die medizinischen Instrumente mit einem weiteren diesbezüglichen Mittel, welches jedoch keine nicht zerfallenden Bestandteile enthält, behandelt werden und wobei der Anteil am Desinfektionsmittel in dem weiteren Mittel geringer ist als der Anteil an Desinfektionsmittel in dem ersten Mittel und wobei das chemische Desinfektionsmittel ausgewählt ist aus organischen Persäuren oder Mischungen derselben.

2. Verwendung nach Anspruch 1, **dadurch gekennzeichnet, dass** die genannten organischen Persäuren ausgewählt sind aus
a) den Persäuren oder Salzen von Persäuren mit der allgemeinen Formel I
R²-O₂C-(CH₂)ₓ-CO₃H (I)
worin R² Wasserstoff oder eine Alkylgruppe von 1 bis 4 Kohlenstoffatomen und ₓ eine Zahl von 1 bis 4 ist, und/oder
b) den Phthalimido-Percarbonsäuren (II), worin der Percarbonsäure-Anteil 1 bis 18 Kohlenstoffatome enthält, und/oder
c) den Verbindungen der Formel III
R¹ - CO₃H (III)
worin R¹ eine Alkyl- oder Alkenylgruppe mit 1 bis 18 Kohlenstoffatomen ist.

3. Verwendung nach Anspruch 2, **dadurch gekennzeichnet, dass**
a) als Persäuren gemäß der allgemeinen Formel I Persäuren enthalten sind, in denen R² Wasserstoff oder eine Methylgruppe ist, und/oder
b) als Persäuren Phthalimido-Persäuren enthalten sind, in denen der Percarbonsäure-Anteil 1 bis 8 Kohlenstoffe enthält, und/oder
c) als Persäuren gemäß der allgemeine Formel III Persäuren mit einer Alkyl- oder Alkenylgruppe mit 1 bis 12 Kohlestoffatomen enthalten sind.

4. Verwendung nach Anspruch 3, **dadurch gekennzeichnet, dass** als Persäuren eine oder mehrere Verbindungen ausgewählt aus Peressigsäure, Perpropionsäure, Peroctansäure, Phthalimidoperhexansäure, Phthalimidoperoctansäure, Perglutarsäure, Perglutarsäuremonomethylester, Peradipinsäure, Peradipinsäuremonomethylester, Perbemsteinsäure, Perbernsteinsäuremonomethylester, enthalten sind.

5. Verwendung nach Anspruch 4, **dadurch gekennzeichnet, dass** als Persäure Peressigsäure enthalten ist.

6. Verwendung nach einem oder mehreren der Ansprüche 1 bis 5, **dadurch gekennzeichnet, dass** die durch UV-Strahlung zu behandelnde Lösung keine nicht zerfallenden Bestandteile enthält.

## Claims

1. Use of an agent obtainable by artificial irradiation with ultraviolet light of an aqueous solution containing 0.0001 to 0.005 weight-%, based on the entire solution, of a chemical disinfectant for a sterilizing treatment of medical instruments without rinsing with pure sterile water, wherein
a) said medical instruments are decontaminated with a first agent according to the above definition, followed by
b) said medical instruments being treated with another respective agent, which, however, does not contain any non-decomposing components, and wherein the percentage of disinfectant in the other agent is lower than the percentage of disinfectant in the first agent, and wherein the chemical disinfectant is selected from organic peroxy acids or mixtures thereof.

2. The use according to claim 1, **characterized in that** said organic peroxy acids are selected among
a) peroxy acids, or salts thereof, of the general formula I:
R²-O₂-C-(CH₂)ₓ-CO₃H (I),
where R² represents hydrogen or an alkyl group having 1 to 4 carbon atoms, and x is a number from 1 to 4, and/or
b) phthalimido peroxycarboxylic acids (II), where the peroxycarboxylic moiety has 1 to 18 carbon atoms, and/or
c) compounds of formula (III):
R¹-CO₃H (III),
where R¹ represents an alkyl or alkenyl group having 1 to 18 carbon atoms.

3. The use according to claim 2, **characterized in that**
a) the peroxy acids of the general formula I present are peroxy acids where R² represents hydrogen or a methyl group, and/or
b) the peroxy acids present are phtalimido peroxy acids where the peroxycarboxylic acid moiety has 1 to 8 carbon atoms, and/or
c) the peroxy acids of the general formula III present are peroxy acids with an alkyl or alkenyl group having 1 to 12 carbon atoms.

4. The use according to claim 3, **characterized in that** said peroxy acids are one or more compounds selected among peracetic acid, perpropionic acid, peroctanoic acid, phthalimido perhexanoic acid, phthalimido peroctanoic acid, perglutaric acid, perglutaric acid monomethyl ester, peradipic acid, peradipic acid monomethyl ester, persuccinic acid, and persuccinic acid monomethyl ester.

5. The use according to claim 4, **characterized in that** a peroxy acid present is peracetic acid.

6. The use according to one or more of the claims 1 to 5, **characterized in that** the solution to be treated by ultraviolet radiation does not contain any non-decomposing components.

## Revendications

1. Utilisation d'un agent pouvant être obtenu par irradiation artificielle aux UV d'une solution aqueuse, qui comprend 0,0001 à 0,005 % en poids d'agent de désinfection chimique par rapport à la solution totale, pour le traitement par stérilisation d'instruments médicaux, en renonçant au rinçage avec de l'eau pure stérile, dans laquelle
a) les instruments médicaux sont décontaminés dans un premier agent selon la définition précédente, et ensuite
b) les instruments médicaux sont traités avec un autre agent y afférent, qui ne comprend cependant aucun ingrédient putrescible, et dans laquelle la proportion d'agent de désinfection dans l'autre agent est inférieure à la proportion d'agent de désinfection dans le premier agent et dans laquelle l'agent de désinfection chimique est choisi parmi les peracides organiques ou des mélanges de ceux-ci.

2. Utilisation selon la revendication 1, **caractérisée en ce que** les peracides organiques cités sont choisis parmi
a) les peracides ou sels de peracides de formule générale I
R²-O₂C-(CH₂) ₓ-CO₃H (I)
dans laquelle R² représente un atome d'hydrogène ou un groupe alkyle ayant de 1 à 4 atomes de carbone et ₓ représente un nombre compris entre 1 et 4, et/ou
b) les peracides phtalamido-carboxyliques (II), dans lesquels la fraction peracide carboxylique comprend 1 à 18 atomes de carbone, et/ou
c) les composés de formule III
R¹-CO₃H (III)
dans laquelle R¹ est un groupe alkyle ou alcényle ayant de 1 à 18 atomes de carbone.

3. Utilisation selon la revendication 2, **caractérisée en ce que**
a) des peracides dans lesquels R² représente un atome d'hydrogène ou un groupe méthyle sont présents à titre de peracides selon la formule générale I, et/ou
b) des peracides phtalimido dans lesquels la fraction peracide carboxylique comprend de 1 à 8 atomes de carbone sont présents à titre de peracides, et/ou
c) des peracides ayant un groupe alkyle ou alcényle comprenant de 1 à 12 atomes de carbone sont présents à titre de peracides selon la formule générale III.

4. Utilisation selon la revendication 3, **caractérisée en ce que** un ou plusieurs composés choisis parmi le peracide acétique, le peracide propionique, le peracide octanoïque, le peracide phtalimido-hexanoïque, le peracide phtalimido-octanoïque, le peracide glutarique, l'ester monométhylique de peracide glutarique, le peracide adipique, l'ester monométhylique de peracide adipique, le peracide succinique, l'ester monométhylique de peracide succinique.

5. Utilisation selon la revendication 4, **caractérisée en ce que** le peracide acétique est présent à titre de peracide.

6. Utilisation selon l'une ou plusieurs des revendications 1 à 5, **caractérisée en ce que** la solution à traiter par le rayonnement UV ne comprend pas d'ingrédients putrescibles.
